# EUROPEAN PATENT SPECIFICATION

(11) **EP 2 956 197 B1**
(45) Date of publication and mention of the grant of the patent: **08.02.2017**
(21) Application number: 14732682.1
(22) Date of filing: 07.05.2014
(51) Int. Cl.: A61M 25/00, A61M 25/06, A61M 25/01

(54) **STEERABLE MEDICAL DEVICE HAVING MULTIPLE CURVE PROFILES**
LENKBARE MEDIZINISCHE VORRICHTUNG MIT MEHREREN KURVENPROFILEN
DISPOSITIF MÉDICAL ORIENTABLE DOTÉ DE MULTIPLES PROFILS DE COURBURE

(30) Priority: 07.05.2013 US 201361820354 P
(43) Date of publication of application: 23.12.2015
(73) Proprietor: St. Jude Medical, Atrial Fibrillation Division, Inc., St. Paul, Minnesota 55117-9913 (US)
(72) Inventor: HEISEL, Jennifer M., Princeton, Minnesota 55371 (US); DAKIN, Gregory J., Edina, Minnesota 55424 (US); MOM, Somally, Savage, Minnesota 55378 (US)
(74) Representative: Kramer Barske Schmidtchen Patentanwälte PartG mbB
(86) International application number: PCT/US2014/037124
(87) International publication number: WO 2014/182797

(56) References cited:
- EP-A1- 0 839 547
- EP-A1- 2 465 568
- US-A1- 2008 091 169

## Description

### BACKGROUND

The present disclosure is generally related to medical devices usable in the human body. More particularly, the present disclosure is directed to steerable introducers, guiding catheters, or other medical devices capable of facilitating delivery of another medical device therethrough, that utilize both fixed and steerable shaft deflections.

Catheters are used for an ever-growing number of procedures. For example, catheters are used for diagnostic, therapeutic, and ablative procedures, to name just a few examples. Typically, the catheter is manipulated through the patient's vasculature and to the intended site, for example, a site within the patient's heart. The catheter typically carries one or more electrodes, which may be used for ablation, diagnosis, or the like.

To facilitate placement of a catheter or other medical device at a location of interest within the patient, it may be introduced through another catheter, often referred to as a "guiding catheter," "introducer catheter," "introducer," "sheath," or the like, and the terms may be used interchangeably herein. Generally speaking, an introducer refers to a tube that may be used to place other catheters or medical devices into specific areas of the body. In some bases, the introducers may be steerable, and used to place catheters and/or other medical devices that have little or no directional control, into specific areas of the patient's body.

In the field of cardiac ablation, for example, introducers may be used to negotiate the patient's vasculature such that an ablation device may be passed therethrough and positioned to ablate arrhythmia-causing cardiac tissue. The introducer catheter itself may be advanced over a guide wire.

Generally, it is known that the introducer catheter must have an overall diameter small enough to negotiate blood vessels while retaining an inner diameter (or "bore size") large enough to accommodate the ablation device therethrough. Furthermore, since the path within the patient is often long and tortuous, steering forces must be transmitted over relatively great distances.

Additionally, certain anatomical targets may be difficult to reach, or to reach in the proper orientation to perform the desired procedure.

US 2008/0091169 A1 discloses a catheter assembly comprising an inner liner made of flexible material and an outer layer having a steering mechanism, the steering mechanism comprising at least one flat wire, and a corresponding lumen for each of the at least one flat wire through which the flat wire may travel, wherein the outer layer comprises a braided wire assembly that includes at least two flat wires braided onto a wire mesh.

EP 0 839 547 A1 discloses a steerable catheter, comprising a preshaped distal section, a flexible intermediate section having a distal end connected to said distal section, a complexly curved proximal section having a distal end connected to said flexible intermediate section, said proximal section being shaped to seat the catheter relative to an anatomical feature within a patient, and means for steering the distal section of the seated catheter into contact with tissue within the patient by flexing said flexible intermediate section.

### BRIEF SUMMARY

Disclosed herein is a method of manufacturing an introducer according to claim 1. In embodiments, the braided wire reinforcing assembly is placed over the inner liner and the steering assembly.

In certain aspects, a layer of a melt-processing polymer is placed between a proximal section of the inner liner and a proximal section of the braided wire reinforcing assembly. This layer can be a polyether block amide.

The outer layer can also include a natural polyether block amide segment. The use of a natural polyether block amide segment in the outer layer more easily allows a proximal end of the steering wire to be pulled through an interstitial space in the braided wire reinforcing assembly, such that a first, more distal portion of the steering wire is radially inside the braided wire reinforcing assembly and a second, more proximal portion of the steering wire is radially outside the braided wire reinforcing assembly.

The fixed curvature can be between 45 degrees and 115 degrees. It can also be in a different plane than the plane in which the steering assembly is operable to deflect the introducer.

Also disclosed herein is a medical device according to claim 10. It is contemplated that the pull wire can terminate in the distal region of the shaft distally of the fixed curvature, for example at a pull ring, such that the steerable region is distal of the fixed curvature region. A handle can be coupled to a proximal end of the shaft, and the handle can further include an actuator coupled to the pull wire of the steering assembly.

In certain embodiments, the shaft also includes a braided wire reinforcing assembly. The pull wire can pass through an interstitial space in the braided wire reinforcing assembly such that a first, more distal portion of the pull wire is radially inside the braided wire reinforcing assembly and a second, more proximal portion of the pull wire is radially outside the braided wire reinforcing assembly.

According to aspects disclosed herein, the shaft has a proximal region that includes a segment of a natural polyether block amide positioned radially outside the braided wire assembly.

It is also contemplated that the steerable region of the shaft is steerable in a first plane and the fixed curvature region of the shaft curves in a second plane different from the first plane.

The foregoing and other aspects, features, details, utilities, and advantages of the present invention will be apparent from reading the following description and claims, and from reviewing the accompanying drawings.

### BRIEF DESCRIPTION OF THE DRAWINGS

FIG. 1 is perspective view of an embodiment of an introducer or guiding catheter in which the principles described herein may be implemented.
FIG. 2 illustrates a perspective view of a section of an introducer according to one embodiment, cut away to show details.
FIG. 3 is a representative cross-sectional view taken along line 3-3 in FIG. 2.
FIG. 4 is a representative cross-sectional view taken along line 4-4 in FIG. 2.
FIG. 5 is a representative cross-sectional view taken along line 5-5 in FIG. 2.
FIG. 6 is a representative cross-sectional view of an introducer assembly prior to the application of heat to melt process the outer layer.
FIG. 7 is a representative cross-sectional view of an introducer after the application of heat to melt process the outer layer.
FIG. 8 illustrates a perspective view of a partially assembled representative introducer in accordance with another embodiment, cut away to show details.
FIG. 9 illustrates a representative pull ring that may be used in an introducer in which the principles described herein may be implemented.
FIG. 10 is a representative cross-sectional view of the pull ring of FIG. 9 taken along line 10-10.

### DETAILED DESCRIPTION

Disclosed herein are various catheters. Catheters according to the instant disclosure generally include both a fixed curve region and a steerable region to facilitate the positioning of the catheter at a desired location, such as a location within a patient's heart. Typically, the fixed curve region will be more proximally located than the steerable region, though the opposite arrangement is also contemplated.

For purposes of explanation, the example of an introducer or guiding catheter will be used throughout. It should be understood, however, that the present teachings can be applied to good advantage in other contexts as well.

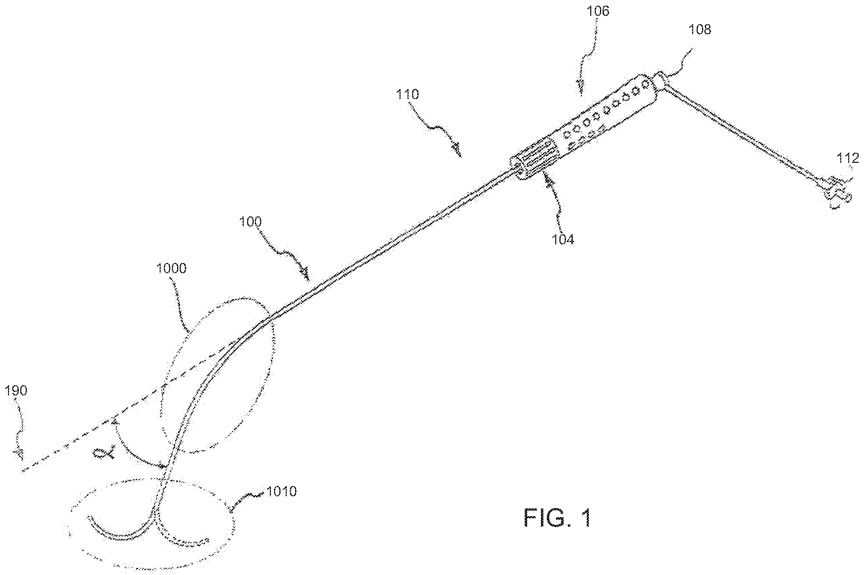

FIG. 1 is a perspective view of an embodiment of an introducer or guiding catheter 1, including a shaft 100 having a proximal portion 110 and a distal portion 190. Shaft 100 may be operably connected to a handle assembly 106, which assists in guiding or steering shaft 100 during diagnostic, therapeutic, and/or other procedures conducted, for example, in a patient's heart. Various handle assemblies suitable for use in connection with the teachings herein will be familiar to those of ordinary skill in the art.

Introducer 1 can also include a hub 108 operably connected to an inner lumen (not shown) within the handle assembly 106 for insertion or delivery of additional medical devices, fluids, or any other devices known to those of ordinary skill in the art. Hub 108 can, for example, be a luer connection, a self-sealing hemostasis valve, a Tuohy Borst valve, or a combination thereof. The inner lumen of handle assembly 106 can also be in communication with the lumen 80 of shaft 100, which is described in further detail below. In representative embodiments, introducer 1 can further include a valve 112 *(e.g.,* a stopcock) operably connected to the hub 108.

FIG. 1 also depicts a proximal fixed curve 1000 and a distal steerable region 1010, both of which are discussed in further detail below. It is contemplated, however, that the arrangement of the fixed curve and steerable regions could be reversed without departing from the scope of the instant disclosure.

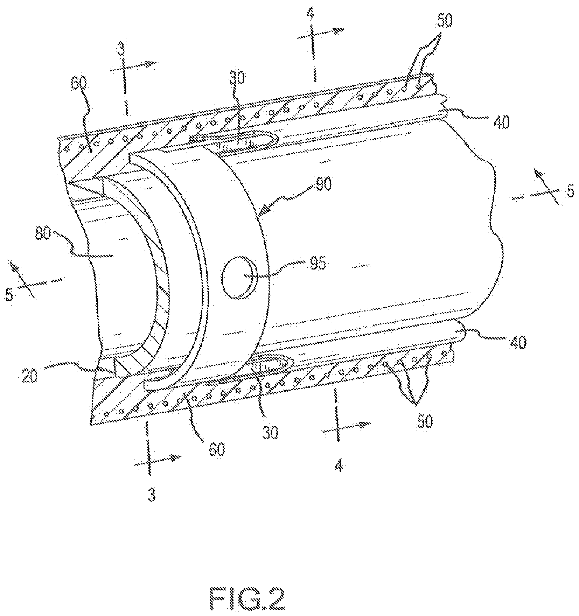

FIG. 2 illustrates a perspective view of a section of shaft 100 according to one embodiment, cut away to show details. Although the details of the construction of shaft 100 will be generally familiar to those of ordinary skill in the art (*see, e.g.,* United States application no. 12/861,555), certain details of the construction will be described herein in order to aid in understanding of the present invention, and one representative method of manufacture of shaft 100 will be described with reference to FIGS. 2-8. As they are assembled, the various components will be collectively referred to as a shaft assembly 200.

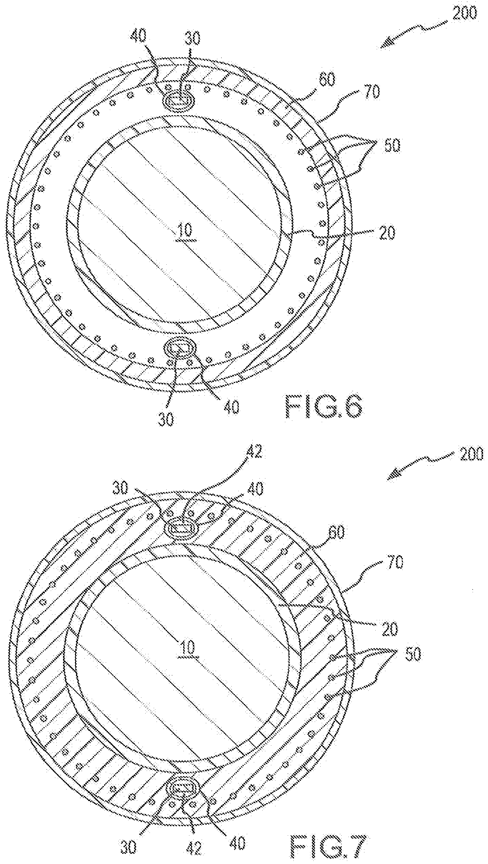

As depicted in FIG. 6, a mandrel 10 is a component of shaft assembly 200, and may be the first component thereof during manufacture of shaft 100. An inner liner 20 is placed on mandrel 10. Inner liner 20 may be knotted at one end *(e.g.,* the distal end) and then fed onto mandrel 10. Of course, inner liner 20 may also be formed about mandrel 10 by any other suitable method.

Inner liner 20 can be an extruded polytetrafluoroethylene (PTFE) tubing, such as TEFLON® brand tubing, which is available commercially. Inner liner 20 may also be made of other melt processing polymers, including, without limitation, etched polytetrafluoroethylene, polyether block amides, nylon and other thermoplastic elastomers. Once such elastomer is PEBAX®, made by Arkema, Inc. PEBAX of various durometers may be used, including, without limitation, PEBAX 30D to PEBAX 72D. In one embodiment, inner liner 20 is made of a material with a melting temperature higher than that of an outer layer 60, which will be further described below, such that inner liner 20 will withstand melt processing of the various components of shaft assembly 200 into shaft 100 as described herein.

Inner liner 20 may be unitary (that is, of a single material). Alternatively, inner liner 20 may be made up of various segments of differing material, and these segments can be longitudinally (that is, abutting one another along the length of mandrel 10) and/or radially (that is, overlapping each other in a manner resembling a bullseye, whether concentrically or eccentrically) arranged.

A steering assembly, including one or more steering wires 30 (also known as "pull wires") and one or more pull rings 90 (discussed in further detail below), can be placed about inner liner 20. Typically, steering wires 30 are arranged substantially longitudinally along inner liner 20. For example, a stainless steel flat wire, such as shown in the accompanying Figures, can be used.

In certain embodiments, at least a portion of steering wire 30 can be encased inside another preformed tube 40 before placement along inner liner 20 to form a lumen 42. Preformed tube 40 need not have the same shape as the cross-section of steering wire 30, but instead may be round, oval, rectangular, or another like shape. Indeed, when preformed tube 40 has a cross-section that is not the same shape as the cross-section of steering wire 30, it facilitates movement of steering wire 30 in preformed tube 40 during manipulations of introducer 1 (*e.g*., deflections of the steerable region, as described in further detail below).

Preformed tube 40 may be formed of polytetrafluoroethylene, polyether block amides, nylon, other thermoplastic elastomers, or another substance. Like inner liner 20, preformed tube 40 can have a higher melting point than outer layer 60 so that preformed tube 40 will not melt when shaft assembly 200 is subjected to melt processing.

In alternative embodiments, steering wire 30 may be covered with lubricious materials including silicone, TEFLON® (PTFE), siloxane, and other lubricious materials before placement. Alternatively, steering wire 30 may also be coated with a lubricious layer to promote slideability. It is also contemplated that steering wire 30 may be manufactured with a smooth surface to promote slideability. While stainless steel is one suitable material from which to compose steering wire 30, other materials may be used; these materials will be generally familiar to those of ordinary skill in the art.

More than one steering wire 30 may also be used. In such cases, each such steering wire 30 can be encased inside its own flexible tube 40 to form separate lumens 42. According to certain aspects, a pair of steering wires 30 are used, spaced apart about 180 degrees about the circumference of inner liner 20.

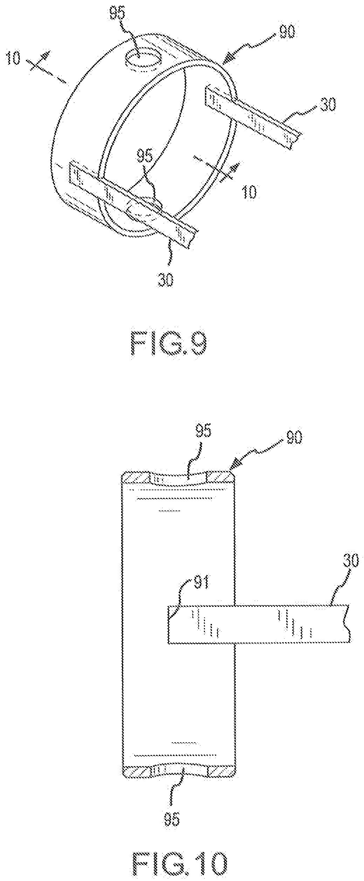

Pull ring 90 provides steerability to shaft 100, e.g., within distal steerable region 1010 shown in FIG. 1. FIGS. 9 and 10 illustrate a representative embodiment for pull ring 90. Pull ring 90 is a generally circular band with a cross-sectional shape (measured orthogonally to a tangential line relative to the circle of the band) that is substantially rectangular. The rectangular cross-section is more clearly depicted in FIG. 10. The outer dimension of pull ring 90 can be determined based on the application for shaft 100 to be manufactured.

Pull ring 90 can have at least one slot 91 that is configured to accommodate steering wire 30. Steering wire 30 may be secured within slot 91 by any technique that is appropriate given the materials of pull ring 90 and steering wires 30. Acceptable techniques may include, but are not limited to, laser welding and/or other welding and bonding techniques.

In another embodiment, pull ring 90 may contain one or more flow holes 95 as illustrated in FIGS. 9 and 10. During a melting process *(e.g.,* during reflow of shaft assembly 200 into shaft 100), the material of outer layer 60 melts and flows through flow holes 95. Upon cooling, the material of outer layer 60 bonds to pull ring 90 to provide better adhesion between pull ring 90 and the remaining components of shaft assembly 200, thereby improving performance of shaft 100. While flow holes 95 are depicted as circular, other shapes may be used. The size and shape of flow holes 95 may be adjusted based on the materials being used to form inner liner 20 and/or outer layer 60.

Pull ring 90 is typically utilized near distal end 190 of shaft 100, but it is contemplated that pull ring 90 may be located at any position along shaft 100. Moreover, more than one pull ring 90 may be utilized in the same shaft 100. In one embodiment of shaft 100, two separate pull rings 90 may be utilized, each of which has its own steering wires 30 connected thereto. Of course, the ordinarily skilled artisan will appreciate that the steerability of shaft 100 can be controlled via the positioning of pull ring(s) 90 therealong. That is, the skilled artisan will appreciate that the manner in which steerable region 1010 is able to be steered will be dependent, in part, upon the number, configuration, and placement of steering wire(s) 30 and pull ring(s) 90.

Outer layer 60 is then placed over inner liner 20, steering wires 30, and (if present) preformed tube 40 forming lumen 42. Outer layer 60 may be made of either single or multiple sections of tubing that may be either butted together or overlapped with each other. Outer layer 60 can be extruded polytetrafluoroethylene tubing, such as TEFLON® brand tubing, which is available commercially. Outer layer 60 may also be made of other melt processing polymers, including, without limitation, etched polytetrafluoroethylene, polyether block amides, nylon and other thermoplastic elastomers. Once such elastomer is PEBAX® made by Arkema, Inc. PEBAX of various durometers may be used, including, without limitation, PEBAX 30D to PEBAX 72D.

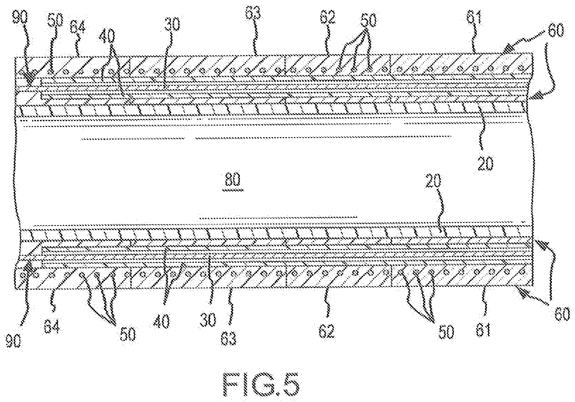

As with inner liner 20, outer layer 60 can include more than one layer and/or more than one segment, including for example two or more segments of the same or differing materials, which can be arranged longitudinally and/or radially to form the entirety of outer layer 60. For example, FIG. 5 illustrates an embodiment in which outer layer 60 includes multiple segments 61, 62, 63, 64, each of which can have different material properties, such as degree of hardness, stiffness, or tensile strength. The length of the segments can also vary.

According to certain aspects, the durometer hardness level of outer layer 60 decreases from proximal end 110 to distal end 190 of shaft 100, thereby minimizing the potential for tissue trauma, with the various segments being reflowed together during manufacturing. Durometer harness levels can also be varied to lend more or less flexibility to, and thus vary the steering/deflection response of, certain segments (*e.g*., lower durometer hardness segments will exhibit a greater degree of deflection for a given force applied to steering wires 30). It should be understood that the number of segments, their hardness levels, and their relative lengths may be adjusted for specific applications, for example to optimize stability and torque delivery for a specific application.

A braided wire assembly 50 is placed over inner liner 20 and steering wires 30 before outer layer 60 is applied. Braided wire assembly 50 may be formed of stainless steel wire, including for example 0.003" round high tensile stainless steel wire. Braided wire assembly 50 can use round wire, flat wire, or combinations thereof.

Braided wire assembly 50 may be formed in a standard braid pattern and uniform density or, alternatively, in a varying braid pattern and/or density. Variations in the braid density of braided wire assembly 50 may be used to increase or decrease flexibility of shaft 100, or of various segments thereof, depending on the desired application. In one embodiment, braided wire assembly 50 has a substantially constant braid density of about 18 to about 21 PPI, which reduces scrap and cost. The ordinarily skilled artisan will appreciate from this disclosure that the braid density can also be varied in light of the diameter of shaft 100, the size of the wires used in braided wire assembly 50, and/or the number of wires used in braided wire assembly 50.

Braided wire assembly 50 may be formed separately on a mandrel or disposable core. One or more portions of braided wire assembly 50 may be heat tempered and cooled before incorporation into shaft assembly 200 through methods that are known to those of ordinary skill. The action of heat tempering may help to release the stress on the wire and help reduce radial forces.

In some embodiments, a layer of a polyether block amide (e.g., PEBAX), or another melt-processing polymer can be included between inner liner 20 and braided wire assembly 50 at the proximal end of shaft assembly 200 (*e.g*., where steering wires 30 exit shaft 100 for attachment to handle assembly 106). The inclusion of this additional layer increases the structural integrity of shaft 100 by protecting inner liner 20 from abrasion or puncture at the location where steering wires 30 are pulled through the braided wire assembly 50 and outer layer 60 prior to attachment to handle assembly 106.

Visibility of steering wires 30 and braided wire assembly 50 can be improved by including a short section of natural (*e.g.,* clear) polyether block amide *(e.g.,* PEBAX) as at least part of outer layer 60 (*e.g.,* as a longitudinally arranged segment of outer layer 60). This clear section simplifies the process of extracting steering wires 30 through the braided wire assembly 50 and the outer layer 60, which in turn reduces the risk of damaging shaft 100 during assembly.

FIG. 6 displays a cross-section of shaft assembly 200 having two steering wires 30 and braided wired assembly 50 encompassed by outer layer 60 before lamination of the materials by heating. In one embodiment, a layer of heat shrink 70 is placed over the top of outer layer 60 as depicted in FIG. 6. Heat shrink 70 is preferably a fluoropolymer or polyolefin material.

FIG. 7 depicts shaft assembly 200 after a lamination process. Shaft assembly 200 may be laminated by heating shaft assembly 200 until the material comprising outer layer 60 flows and redistributes around the circumference thereof as depicted in FIG. 7. Heat shrink 70 has a higher melting temperature than outer layer 60; during the melt process, heat shrink 70 retains its tubular shape and forces the liquefied outer layer 60 material into braided wire assembly 50 (if present) and into contact, for example, with preformed tubes 40 around steering wires 30 and inner liner 20. Shaft assembly 200 may then be cooled, with mandrel 10 still in place, thus maintaining the inner diameter and shape of shaft assembly 200.

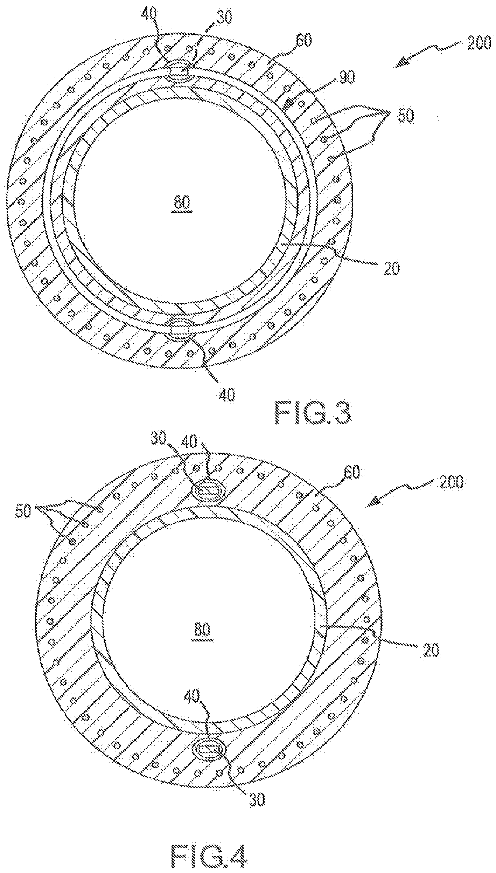

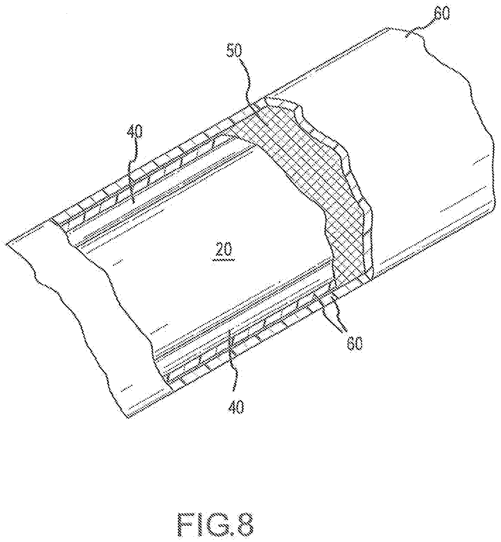

Mandrel 10 may be removed from shaft assembly 200, leaving behind reflowed outer layer 60 and an inner lumen 80 as illustrated in FIGS. 4 and 8, which depict a shaft 100 made in accordance with the representative method described above subsequent to the application of heat for the lamination process. In other embodiments, heat shrink 70 may be left in place around outer layer 60, as depicted in FIG. 7, even after mandrel 10 is removed.

If heat shrink 70 is removed, outer layer 60 becomes the outermost layer of shaft 100. The result of the reflow process described above is a substantially circular shaft 100 with pull wires 30 embedded within outer layer material as illustrated in FIGS. 3 and 4. FIG. 3 is a cross-sectional view taken at the point of a pull ring 90 as depicted in FIG. 2, while FIG. 4 is a cross-sectional view taken at a point proximal to pull ring 90. FIG. 8 is a perspective view of catheter shaft 200, cut away to show certain details of construction.

Shaft assembly 200 may be manufactured using alternative techniques. In one embodiment, outer layer 60 may be formed by extruding outer layer 60 over inner layer 20, braided wire assembly 50, and the like during the buildup of shaft assembly 200. In another embodiment, shaft assembly 200 may formed by using a combination of heat and a press that has a mold for defining the final shape of shaft 100.

Shaft 100 formed using the methods disclosed herein, and in particular lumen 80, may have varying sizes and various uses. Particularly desirable lumen sizes for use as an introducer include, for example, between about 6F and about 24F, or between about 12F and about 14F. These and other bore sizes are feasible. Indeed, by using flat wires to form braided wire assembly 50, one can achieve very thin-walled shafts 100, allowing for larger lumen sizes for given outside dimensions.

Proximal fixed curve 1000, shown in FIG. 1, can also be fixed during manufacture of shaft 100. For example, proximal fixed curve 1000 can be formed by thermosetting shaft 100 into a desired curvature or by including a relatively rigid, pre-shaped tendon or stylet as part of the construction of shaft 100.

Various fixed curves 1000 can be utilized, depending on the particular application of shaft 100. Fixed curves 1000 can be described with reference to the angle (α) they form with the longitudinal axis of shaft 100 and their radius of curvature. An "acute" fixed curve has a smaller radius of curvature than a "gradual" fixed curve. Stated another way, an "acute" fixed curve occurs over a shorter length of shaft 100 than does a "gradual" fixed curve. Thus, for example, a fixed curve 1000 can be 45 degrees acute (*e.g*., a relatively sharp 45 degree bend) or 45 degrees gradual *(e.g.,* a more relaxed 45 degree bend). Another exemplary fixed curve 1000 is 115 degrees gradual.

The combination of fixed curve region 1000 and steerable region 1010 facilitates the placement of distal end 190 of shaft 100, as part of introducer 1, at a desired location within a patient, such as the left atrial appendage. The ordinarily skilled artisan will understand from the instant disclosure that the fixed curve of region 1000 causes the distal end 190 of shaft 100 to be positioned generally in the vicinity of the desired location, while the steerability of region 1010 allows the practitioner to "fine tune" the position of distal end 190 of shaft 100. Thus, a medical device, such as a left atrial appendage closure device (*e.g*., the AMPLATZER™ Amulet™ left atrial appendage occluder of St. Jude Medical, Inc.), is reliably delivered to the target location.

Fixed curve region 1000 and steerable region 1010 can be in the same or different planes. Thus, for example, fixed curve region 1000 can curve shaft 100 within a first plane (e.g., the X-Y plane), while steerable region 1010 is steerable in a second plane, which can be perpendicular to the first plane (e.g., the X-Z or Y-Z plane).

The present disclosure also contemplates the inclusion of a tip assembly for use in medical procedures, such as an atraumatic tip, including, for example, a radiopaque material contained therein for location of the tip during use. The tip assembly can be configured with a plurality of port holes for delivery of, for example, radiopaque contrast or irrigation fluid, with ablation electrodes for use in cardiac ablation procedures, with mapping electrodes for use in electrophysiology studies, or with any other desirable structures.

Although several embodiments have been described above with a certain degree of particularity, those skilled in the art could make numerous alterations to the disclosed embodiments without departing from the scope of this invention as defined by the appended claims.

For example, pull ring 90 and braided wire assembly 50 may be made of stainless steel or other materials.

All directional references (e.g., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the scope of the invention as defined in the appended claims.

## Claims

1. A method of manufacturing an introducer (1), comprising:
placing an inner liner (20) over a mandrel (10);
placing a steering assembly over the inner liner (20), the steering assembly including a steering wire (30) and a pull ring (90);
placing a braided wire reinforcing assembly over the inner liner (20);
placing an outer layer (60) over the inner liner (20), the steering assembly, and the braided wire reinforcing assembly;
laminating the outer layer (60) to the inner liner (20), thereby embedding the steering assembly and the braided wire reinforcing assembly within the outer layer (60); and
removing the mandrel (10), thereby forming a lumen (80),
wherein a portion of the introducer (1) is formed into a fixed curvature,
**characterized in that**
a proximal end of the steering wire (30) is pulled through an interstitial space in the braided wire reinforcing assembly, such that a first, more distal portion of the steering wire is radially inside the braided wire reinforcing assembly and a second, more proximal portion of the steering wire is radially outside the braided wire reinforcing assembly.

2. The method according to claim 1, wherein placing the braided wire reinforcing assembly over the inner liner (20) comprises placing the braided wire reinforcing assembly over the inner liner (20) and the steering assembly.

3. The method according to claim 1, wherein the fixed curvature of the introducer (1) is proximal of the pull ring (90).

4. The method according to claim 1, further comprising placing a layer of a melt-processing polymer between a proximal section of the inner liner (20) and a proximal section of the braided wire reinforcing assembly.

5. The method according to claim 4, wherein the layer of a melt-processing polymer between the inner liner (20) and the braided wire reinforcing assembly comprises a polyether block amide.

6. The method according to claim 1, wherein the outer layer (60) comprises a natural polyether block amide segment.

7. The method according to claim 1, wherein the fixed curvature comprises a 45 degree curvature.

8. The method according to claim 1, wherein the fixed curvature comprises a 115 degree curvature.

9. The method according to claim 1, wherein the steering assembly is operable to deflect the introducer (1) in a first plane, and wherein the fixed curvature bends the introducer (1) in a second plane different from the first plane.

10. A medical device, comprising:
a shaft (100) defining a lumen (80) therethrough and having a distal region, wherein the distal region of the shaft (100) includes a fixed curvature region and a steerable region (1010); and
a steering assembly including a pull wire (30), the pull wire (30) terminating in the steerable region (1010) of the shaft (100),
**characterized in that**
the shaft (100) further comprises a braided wire reinforcing assembly, wherein the pull wire (30) passes through an interstitial space in the braided wire reinforcing assembly such that a first, more distal portion of the pull wire (30) is radially inside the braided wire reinforcing assembly and a second, more proximal portion of the pull wire (30) is radially outside the braided wire reinforcing assembly.

11. The medical device according to claim 10, wherein the pull wire (30) terminates in the distal region of the shaft (100) distally of the fixed curvature, such that the steerable region (1010) is distal of the fixed curvature region.

12. The medical device according to claim 10, further comprising a handle coupled to a proximal end of the shaft (100), wherein the handle includes an actuator coupled to the pull wire (30) of the steering assembly.

13. The medical device according to claim 10, wherein the steering assembly comprises a pull ring (90), and wherein the pull wire (30) terminates at the pull ring (90).

14. The medical device according to claim 10, wherein the shaft (100) includes a proximal region, and wherein the proximal region comprises a segment of a natural polyether block amide positioned radially outside the braided wire assembly.

15. The medical device according to claim 10, wherein the steerable region (1010) of the shaft (100) is steerable in a first plane and wherein the fixed curvature region of the shaft (100) curves in a second plane different from the first plane.

## Patentansprüche

1. Verfahren zur Herstellung einer Einführhilfe (1), umfassend:
Anordnen einer inneren Auskleidung (20) auf einem Dorn (10),
Anordnen einer Steueranordnung auf der inneren Auskleidung (20), wobei die Steueranordnung einen Steuerdraht (30) und einen Zugring (90) umfasst,
Anordnen einer Flechtdraht-Verstärkungsanordnung auf der inneren Auskleidung (20),
Anordnen einer Außenschicht (60) auf der inneren Auskleidung (20), der Steueranordnung und der Flechtdraht-Verstärkungsanordnung,
Laminieren der Außenschicht (60) an die innere Auskleidung (20), wodurch die Steueranordnung und die Flechtdraht-Verstärkungsanordnung innerhalb der Außenschicht (60) eingebettet werden, und
Entfernen des Dorns (10), wodurch ein Lumen (80) gebildet wird,
wobei ein Abschnitt der Einführhilfe (1) zu einer feststehenden Krümmung ausgebildet wird,
**dadurch gekennzeichnet, dass**
ein proximales Ende des Steuerdrahts (30) durch einen Zwischenraum in der Flechtdraht-Verstärkungsanordnung gezogen wird, so dass sich ein erster, mehr distaler Abschnitt des Steuerdrahts radial innerhalb der Flechtdraht-Verstärkungsanordnung befindet und sich ein zweiter, mehr proximaler Abschnitt des Steuerdrahts radial außerhalb der FlechtdrahtVerstärkungsanordnung befindet.

2. Verfahren nach Anspruch 1, bei dem das Anordnen der FlechtdrahtVerstärkungsanordnung auf der inneren Auskleidung (20) das Anordnen der FlechtdrahtVerstärkungsanordnung auf der inneren Auskleidung (20) und der Steueranordnung umfasst.

3. Verfahren nach Anspruch 1, bei dem sich die feststehende Krümmung der Einführhilfe (1) proximal von dem Zugring (90) befindet.

4. Verfahren nach Anspruch 1, das ferner das Anordnen einer Schicht aus einem Schmelzverarbeitungspolymer zwischen einem proximalen Abschnitt der inneren Auskleidung (20) und einem proximalen Abschnitt der Flechtdraht-Verstärkungsanordnung umfasst.

5. Verfahren nach Anspruch 4, bei dem die Schicht aus einem Schmelzverarbeitungspolymer zwischen der inneren Auskleidung (20) und der Flechtdraht-Verstärkungsanordnung ein Polyetherblockamid umfasst.

6. Verfahren nach Anspruch 1, bei dem die Außenschicht (60) ein natürliches Polyetherblockamidsegment umfasst.

7. Verfahren nach Anspruch 1, bei dem die feststehende Krümmung eine 45 Grad-Krümmung umfasst.

8. Verfahren nach Anspruch 1, bei dem die feststehende Krümmung eine 115 Grad-Krümmung umfasst.

9. Verfahren nach Anspruch 1, bei dem die Steueranordnung so betrieben werden kann, dass die Einführhilfe (1) in einer ersten Ebene gebogen wird, und wobei die feststehende Krümmung die Einführhilfe (1) in einer zweiten Ebene, die sich von der ersten Ebene unterscheidet, biegt.

10. Medizinische Vorrichtung, umfassend:
einen Schaft (100), der ein Lumen (80) durch diesen festlegt und einen distalen Bereich aufweist, wobei der distale Bereich des Schafts (100) einen feststehenden Krümmungsbereich und einen steuerbaren Bereich (1010) umfasst, und
eine Steueranordnung, die einen Zugdraht (30) umfasst, wobei der Zugdraht (30) in dem steuerbaren Bereich (1010) des Schafts (100) endet,
**dadurch gekennzeichnet, dass**
der Schaft (100) ferner eine Flechtdraht-Verstärkungsanordnung umfasst, wobei der Zugdraht (30) derart durch einen Zwischenraum in der Flechtdraht-Verstärkungsanordnung hindurchtritt, dass sich ein erster, mehr distaler Abschnitt des Zugdrahts (30) radial innerhalb der Flechtdraht-Verstärkungsanordnung befindet und sich ein zweiter, mehr proximaler Abschnitt des Zugdrahts (30) radial außerhalb der Flechtdraht-Verstärkungsanordnung befindet.

11. Medizinische Vorrichtung nach Anspruch 10, bei welcher der Zugdraht (30) in dem distalen Bereich des Schafts (100) distal von der feststehenden Krümmung endet, so dass der steuerbare Bereich (1010) distal von dem Bereich mit feststehender Krümmung vorliegt.

12. Medizinische Vorrichtung nach Anspruch 10, die ferner einen Griff umfasst, der an ein proximales Ende des Schafts (100) gekoppelt ist, wobei der Griff einen Aktuator umfasst, der mit dem Zugdraht (30) der Steueranordnung gekoppelt ist.

13. Medizinische Vorrichtung nach Anspruch 10, welcher die Steueranordnung einen Zugring (90) umfasst und bei welcher der Zugdraht (30) an dem Zugring (90) endet.

14. Medizinische Vorrichtung nach Anspruch 10, bei welcher der Schaft (100) einen proximalen Bereich umfasst und wobei der proximale Bereich ein Segment aus einem natürlichen Polyetherblockamid umfasst, das radial außerhalb der Flechtdrahtanordnung angeordnet ist.

15. Medizinische Vorrichtung nach Anspruch 10, bei welcher der steuerbare Bereich (1010) des Schafts (100) in einer ersten Ebene steuerbar ist und bei welcher der Bereich mit feststehender Krümmung des Schafts (100) in einer zweiten Ebene gekrümmt ist, die von der ersten Ebene verschieden ist.

## Revendications

1. Procédé de fabrication d'un introducteur (1), comprenant les étapes suivantes:
placer une doublure intérieure (20) sur un mandrin (10);
placer un ensemble de direction sur la doublure intérieure (20), l'ensemble de direction comprenant un fil de direction (30) et un anneau de traction (90);
placer un ensemble de renforcement de fil tressé sur la doublure intérieure (20);
placer une couche extérieure (60) sur la doublure intérieure (20), l'ensemble de direction et l'ensemble de renforcement de fil tressé;
stratifier la couche extérieure (60) sur la couche intérieure (20), incorporant de ce fait l'ensemble de direction et l'ensemble de renforcement de fil tressé à l'intérieur de la couche extérieure (60); et
enlever le mandrin (10), formant de ce fait une lumière (80),
dans lequel une partie de l'introducteur (1) est formée avec une courbure fixe,
**caractérisé en ce qu'**une extrémité proximale de l'ensemble de direction (30) est tirée à travers un espace interstitiel dans l'ensemble de renforcement de fil tressé, de telle sorte qu'une première partie plus distale du fil de direction soit située radialement à l'intérieur de l'ensemble de renforcement de fil tressé, et qu'une seconde partie plus proximale du fil de direction soit située radialement à l'extérieur de l'ensemble de renforcement de fil tressé.

2. Procédé selon la revendication 1, dans lequel le placement de l'ensemble de renforcement de fil tressé sur la doublure intérieure (20) comprend le placement de l'ensemble de renforcement de fil tressé sur la doublure intérieure (20) et l'ensemble de direction.

3. Procédé selon la revendication 1, dans lequel la courbure fixe de l'introducteur (1) est proche de l'anneau de traction (90).

4. Procédé selon la revendication 1, comprenant en outre le placement d'une couche d'un polymère à traiter par fusion entre une section proximale de la doublure intérieure (20) et une section proximale de l'ensemble de renforcement de fil tressé.

5. Procédé selon la revendication 4, dans lequel la couche de polymère à traiter par fusion entre la doublure intérieure (20) et l'ensemble de renforcement de fil tressé comprend un polyéther bloc amide.

6. Procédé selon la revendication 1, dans lequel la couche extérieure (60) comprend un segment de polyéther bloc amide naturel.

7. Procédé selon la revendication 1, dans lequel la courbure fixe comprend une courbure à 45 degrés.

8. Procédé selon la revendication 1, dans lequel la courbure fixe comprend une courbure à 115 degrés.

9. Procédé selon la revendication 1, dans lequel l'ensemble de direction est actionnable pour défléchir l'introducteur (1) dans un premier plan, et dans lequel la courbure fixe plie l'introducteur (1) dans un second plan différent du premier plan.

10. Dispositif médical, comprenant:
un arbre (100) définissant une lumière (80) à travers celui-là et présentant une région distale, dans lequel la région distale de l'arbre (100) comprend une région de courbure fixe et une région dirigeable (1010); et
un ensemble de direction comprenant un fil de traction (30), le fil de traction (30) se terminant dans la région dirigeable (1010) de l'arbre (100),
**caractérisé en ce que** l'arbre (100) comprend en outre un ensemble de renforcement de fil tressé, dans lequel le fil de traction (30) passe à travers un espace interstitiel dans l'ensemble de renforcement de fil tressé, de telle sorte qu'une première partie plus distale du fil de traction (30) soit située radialement à l'intérieur de l'ensemble de renforcement de fil tressé, et qu'une seconde partie plus proximale du fil de traction (30) soit située radialement à l'extérieur de l'ensemble de renforcement de fil tressé.

11. Dispositif médical selon la revendication 10, dans lequel le fil de traction (30) se termine dans la région distale de l'arbre (100) à distance de la courbure fixe, de telle sorte que la région dirigeable (1010) soit distante de la région de courbure fixe.

12. Dispositif médical selon la revendication 10, comprenant en outre une poignée couplée à une extrémité proximale de l'arbre (100), dans lequel la poignée comprend un actionneur couplé au fil de traction (30) de l'ensemble de direction.

13. Dispositif médical selon la revendication 10, dans lequel l'ensemble de direction comprend un anneau de traction (90), et dans lequel le fil de traction (30) se termine au niveau de l'anneau de traction (90).

14. Dispositif médical selon la revendication 10, dans lequel l'arbre (100) comprend une région proximale, et dans lequel la région proximale comprend un segment d'un polyéther bloc amide naturel positionné radialement à l'extérieur de l'ensemble de fil tressé.

15. Dispositif médical selon la revendication 10, dans lequel la région dirigeable (1010) de l'arbre (100) est dirigeable dans un premier plan, et dans lequel la région de courbure fixe de l'arbre (100) s'incurve dans un second plan différent du premier plan.
